# EUROPÄISCHE PATENTSCHRIFT

(11) **EP 0 804 937 B1**
(45) Veröffentlichungstag und Bekanntmachung des Hinweises auf die Patenterteilung: **08.04.1998**
(21) Anmeldenummer: 96106958.0
(22) Anmeldetag: 03.05.1996
(51) Int. Cl.: A61M 25/01

(54) **Verfahren zur Herstellung eines Führungsdrahtes und Führungsdraht**
Guidewire and its manufacturing procedure
Fil de guidage et procédé de sa fabrication

(43) Veröffentlichungstag der Anmeldung: 05.11.1997
(73) Patentinhaber: SCHNEIDER (EUROPE) AG, 8180 Bülach (CH)
(72) Erfinder: Willi Jakob, 8181 Höri (CH)
(74) Vertreter: Groner, Manfred

(56) Entgegenhaltungen:
- EP-A- 0 625 358
- US-A- 4 646 044
- US-A- 4 934 380
- US-A- 5 282 478
- US-A- 5 415 170

## Beschreibung

Die Erfindung betrifft ein Verfahren zur Herstellung eines Führungsdrahtes, insbesondere zum perkutanen Einführen eines Ballondilatationskatheters in ein Blutgefäss, welcher Führungsdraht eine flexible Spirale aufweist, die einen ebenfalls flexiblen Schaft umgibt und aus wenigstens zwei Spiralfedern hergestellt ist, die an einer Verbindungsstelle über mehrere Windungen zusammengedreht und miteinander verlötet sind. Die Erfindung betrifft zudem einen Führungsdraht, insbesondere zum perkutanen Einführen eines Ballondilatationskatheters in ein Blutgefäss mit einer flexiblen Spirale, die einen ebenfalls flexiblen Schaft umgibt und aus wenigstens einer distalen und einer proximalen Spiralfeder hergestellt ist, welche Spiralfedern an einer Verbindungsstelle über mehrere Windungen zusammengedreht und miteinander verlötet sind.

Führungsdrähte der genannten Art sind im Stand der Technik in zahlreichen Ausführungen bekannt und werden insbesondere zum perkutanen Einführen eines Ballondilatationskatheters in ein Blutgefäss insbesondere eine Engstelle eines solchen Gefässes verwendet. Damit der Führungsdraht zum Einführen eines Ballondilatationskatheters in eine Engstelle eines Blutgefässes geeignet ist, muss er ausser einer hohen Funktionssicherheit besondere Eigenschaften aufweisen und insbesondere steuerbar und am distalen Ende sehr flexibel sein. Zudem soll der Führungsdraht einen vergleichsweise kurzen röntgensichtbaren Bereich besitzen, der scharf abgegrenzt ist.

Ein solcher Führungsdraht ist beispielsweise in der US-A-4,748,986 offenbart. Dieser weist eine Spirale auf, die aus einer distalen und röntgensichtbaren Spiralfeder sowie einer proximalen Spiralfeder besteht. Die Spirale umgibt einen Schaft, der am distalen Ende konisch verjüngt und mit einer Spitze verbunden ist. Zum Verbinden der beiden Spiralfedern werden diese an den zu verbindenden Enden über mehrere Windungen zusammengedreht und durchgehend mit dem Schaft sowie einem Sicherheitsband verlötet. Dieses Zusammenfügen der Spiralfedern und das Verlöten ist eine ausserordentliche aufwendige und vor allem zeitintensive Handarbeit. Durch die vergleichsweise massive Lötstelle ist der Führungsdraht an der Verbindungsstelle wesentlich weniger flexibel als vor und nach diesem Bereich. Damit wird die Steuerbarkeit des Führungsdrahtes beeinträchtigt. Zur Verbesserung der gewünschten Eigenschaften des Führungsdrahtes ist in der EP-A-0 625 358 vorgeschlagen, die beiden Spiralfedern mittels einer Verbindungsspirale zu verbinden. Diese vergleichsweise kurze Verbindungsspirale wird nach einem Dehnen der zu verbindenden Windungen sowie dem Einsetzen des flexiblen Schaftes eingedreht und verlötet. Damit wird unter anderem erreicht, dass die Flexibilität des Führungsdrahtes durch die Verbindungsstelle weniger beeinträchtigt wird. Der Zeitaufwand zum Verbinden der beiden Spiralfedern ist aber vergleichsweise hoch, wobei die ausserordentliche Feinheit der zu verbindenden Spiralfedern sowie die hohen Anforderungen an die Zuverlässigkeit der Verbindungsstelle berücksichtigt werden müssen.

Eine weitere Möglichkeit, zwei Spiralfedern zu verbinden, zeigt die EP-A-0 419 277. Bei diesem Verfahren wird ein Zwischenstück verwendet, das aussenseitig Rillen aufweist, auf welche die zu verbindenden Spiralfederenden aufgedreht werden.

Der Erfindung liegt die Aufgabe zugrunde, ein Verfahren und einen Führungsdraht der genannten Gattungen zu schaffen, die eine schnellere und dennoch den hohen Anforderungen der Sicherheit genügende Herstellung ermöglichen.

Das erfindungsgemässe Verfahren ist dadurch gekennzeichnet, dass die beiden Spiralfedern jeweils an dem zu verbindenden Ende auf eine Zentrierform aufgeschoben, zusammengedreht und verlötet werden und dass anschliessend die Zentrierform wieder entfernt wird. Beim erfindungsgemässen Verfahren entfällt das Eindrehen der ausserordentlich feinen Verbindungsspirale und dennoch wird eine Verbindungsstelle geschaffen, die rohrförmig ist und insbesondere bezüglich Flexibilität mit den übrigen Bereichen des Führungsdrahtes vergleichbar ist. Die Zentrierform bildet eine Lehre, welche das Zusammendrehen der beiden Spiralfedern wesentlich erleichtert und damit ein schnelleres und dennoch zuverlässiges Arbeiten ermöglicht. Die beiden zusammengedrehten Spiralfedern werden durch die Zentrierform aufeinander ausgerichtet und können einfach und dennoch präzis verlötet werden. Die Zentrierform verhindert, dass das Lot in das Innere der Spirale eindringen kann, so dass die Verbindungsstelle nach dem Entfernen der Zentrierform innen hohl und glatt ist. Dadurch kann die Spiralfedereinheit vormontiert und erst in einem späteren Arbeitsgang auf den flexiblen Schaft aufgebracht werden. Vormontierte Spiralfedereinheiten können somit an Lager gehalten werden, was die Organisation der Montage wesentlich erleichtert.

Die Zentrierform ist gemäss einer Weiterbildung der Erfindung so ausgebildet, dass diese das Lot während des Verlötens der Spiralfedern nicht annimmt und sich mit diesem nicht verbindet. Eine solche Zentrierform wird vorzugsweise aus einer geeigneten Titan-Legierung, beispielsweise aus Tynel oder NiTi-Legierung hergestellt. Eine solche Zentrierform ist sehr flexibel und es hat sich gezeigt, dass es für die Herstellung einer solchen Verbindung ausserordentlich eignet. Da sich das Lot mit der Zentrierform nicht verbindet und flexibel ist, kann sie nach dem Verlöten sehr einfach und schonend entfernt werden.

Der erfindungsgemässe Führungsdraht ergibt sich aus Anspruch 8. Dieser Führungsdraht wird vorzugsweise nach dem obengenannten Verfahren hergestellt. Er hat den besonderen Vorteil, dass er im Bereich der Verbindungsstelle weitgehend ähnliche Eigenschaften aufweist wie vor und nach dieser Stelle. Vorzugsweise ist das Lot so aufgetragen, dass es im Bereich der Verbindungsstelle die Aussen- und die Innenseite der Spirale nicht oder nicht wesentlich überragt. Damit ist der Bereich der Verbindungsstelle von den übrigen Bereichen bezüglich der meisten Eigenschaften des Führungsdrahtes kaum zu unterscheiden.

Weitere vorteilhafte Merkmale ergeben sich aus den abhängigen Patentansprüchen, der nachfolgenden Beschreibung sowie der Zeichnung.

Ausführungsbeispiele der Erfindung werden nachfolgend anhand der Zeichnung näher erläutert. Es zeigen:
Figur 1 schematisch das Zusammendrehen zweier Spiralfedern,
Figur 2 schematisch zwei zusammengedrehte Spiralfederenden,
Figur 3 schematisch zwei zusammengedrehte und verlötete Spiralfederenden,
Figur 4 schematisch einen Abschnitt eines erfindungsgemässen Führungsdrahtes, und
Figur 5 schematisch einen Abschnitt eines Führungsdrahtes nach einer Variante.

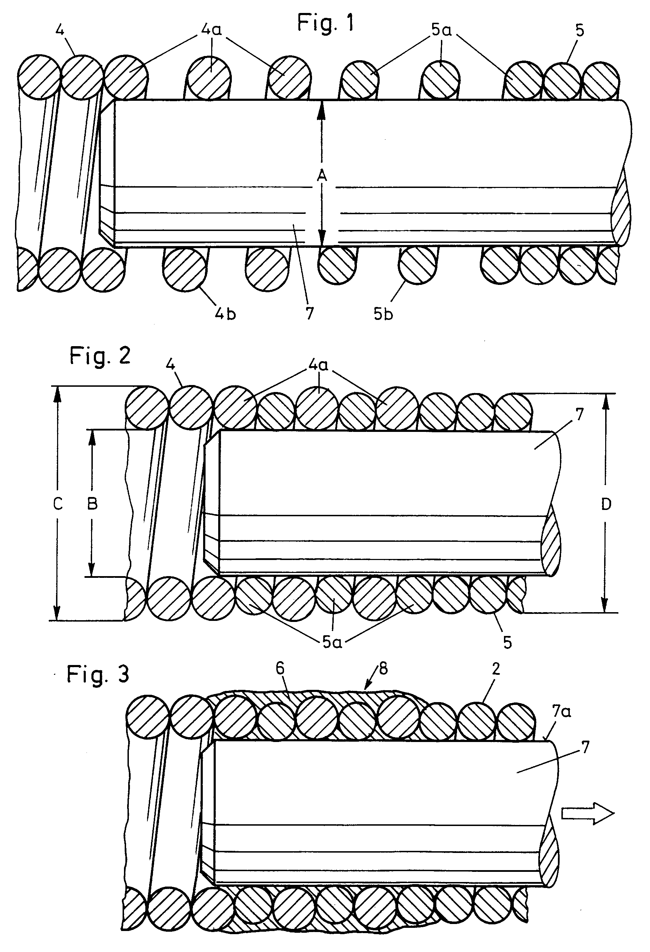

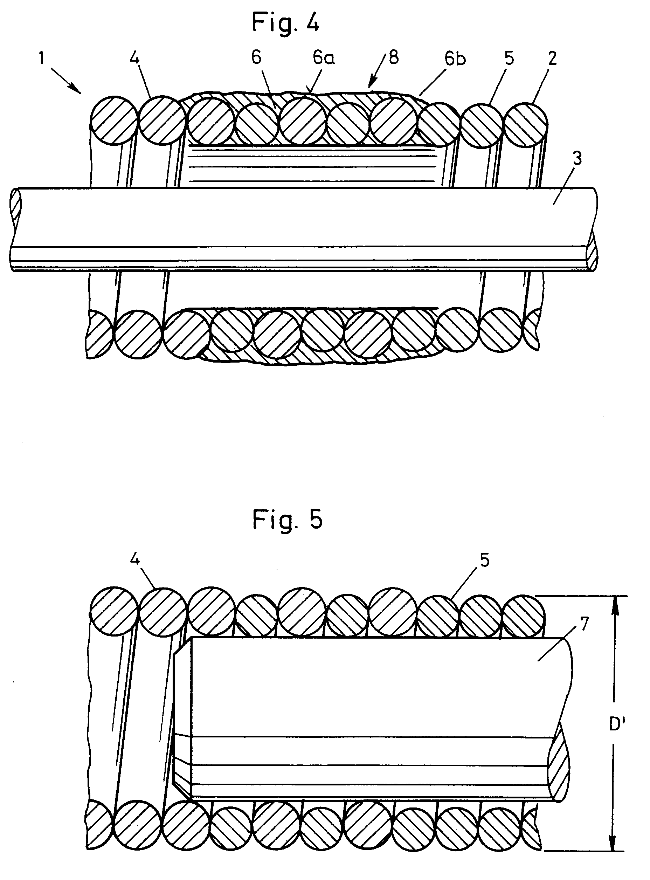

Die Figur 4 zeigt einen Abschnitt eines Führungsdrahtes 1, der mit Ausnahme der hier gezeigten Verbindungsstelle 8 genau demjenigen nach der oben erwähnten EP-A-0 625 358 entsprechen kann. Diese Schrift wird hier für die Offenbarung beigezogen. In Figur 4 ist das hier nicht gezeigte distale Ende rechts und das proximale Ende links. Der Führungsdraht 1 weist einen flexiblen Schaft 3 auf, der am hier nicht gezeigten distalen Ende verjüngt und flachgeschlagen sowie mit der hier ebenfalls nicht gezeigten Spitze fest verbunden ist. Der Schaft 3 bildet einen Kerndraht und ist über seine gesamte Länge von einer ebenfalls flexiblen Spirale 2 umgeben, die eine proximale Spiralfeder 4 und eine distale Spiralfeder 5 aufweist, die an einer Verbindungsstelle 8 fest miteinander verbunden sind. Die Spirale 4 besteht vorzugsweise aus einem rostfreien Draht mit einem Durchmesser von beispielsweise etwa 0,06 mm. Der Aussendurchmesser C (Figur 2) dieser Spiralfeder 4 beträgt vorzugsweise etwa 0,31 - 0,32 mm. Die distale Spiralfeder 5 ist aus einem röntensichtbaren Werkstoff, beispielsweise Wolfram hergestellt und besitzt einen Aussendurchmesser D von vorzugsweise etwa 0,31 mm. Die Spiralfeder 5 ist aus einem Draht gewunden, der einen Durchmesser von beispielsweise etwa 0,05 mm aufweist.

Nachfolgend wird das Verbinden der beiden Spiralfedern 4 und 5 anhand der Figuren 1 bis 3 näher erläutert.

Um die beiden Spiralfedern 4 und 5 miteinander fest zu verbinden, werden an den zu verbindenden Enden einige Windungen, vorzugsweise 2 bis 5 Windungen 4a und 5a geöffnet und zusammengedreht. Hierbei werden die beiden Spiralfedern 4 und 5 vorzugsweise auf eine Zentrierform 7 aufgezogen, die eine Lehre bildet. Der Aussendurchmesser A der Zentrierform 7 ist gleich oder geringfügig kleiner als der Innendurchmesser B der Spiralfedern 4 und 5. Beim Zusammendrehen können die Spiralen infolge der Führung der Zentrierform seitlich nicht weglaufen.

Die Figur 2 zeigt die beiden Spiralfedern 4 und 5 nach dem Zusammendrehen der aufgeweiteten Windungen 4a und 5a. Auf diese Windungen 4a und 5a wird nun gemäss Figur 3 Lot 6 rundum aufgetragen. Dieses Lot dringt in die Zwischenräume dieser Windungen 4a und 5a ein und umschliesst die Zentrierform 7 an seiner glatten und zylindrischen Aussenseite 7a im Bereich der genannten Windungen 4a und 5a. Diese Zentrierform 7 ist nun vorzugsweise so ausgebildet, dass es das verwendete Lot nicht annimmt und sich auch nicht mit diesem verbindet. Vorzugsweise besteht die Zentrierform aus einer Nickel-Titan-Legierung, beispielsweise Tynel oder NiTi-Legierung. Möglich sind jedoch auch andere geeignete Legierungen aus Wolfram oder Molybdän. Diese Legierungen nehmen das Lot 6 nicht an und ergeben ein flexibles Draht- und Rohrstück 7. Nach dem Verlöten kann die Zentrierform 7 ohne Beschädigung der Verbindungsstelle 8 sehr einfach von der Spirale 2 abgezogen werden. Die beiden Spiralfedern 4 und 5 sind nun an der Verbindungsstelle 8 fest miteinander verbunden. Die Innenseite 6d der Verbindungsstelle 8 ist glatt und zylindrisch und zwar korrespondierend der Aussenseite 7a der Zentrierform 7. Die Verbindungsstelle 8 ist ebenfalls zylindrisch und überragt die Aussenseite der Spirale 2 im wesentlichen nicht.

Nach dem Entfernen der Zentrierform 7 wird der Schaft 3 in die Spirale 2 eingesetzt und in bekannter Weise mit der Spitze verbunden. Wesentlich ist nun, dass das Einführen des Schaftes 3 durch die Verbindungsstelle 8 nicht behindert wird, da diese innen ebenso weit ist wie die Spirale 2 vor und nach diesem Bereich. Die Spirale 2 lässt sich somit ohne Schaft 3 vormontieren und an Lager legen.

Wie oben erwähnt, ist die distale Spiralfeder 5 aus einem Draht gewunden, der einen kleineren Durchmesser aufweist, als derjenige der proximalen Spiralfeder 4, was die Zeichnung ebenfalls erkennen lässt. Nach einer in Figur 5 dargestellten Weiterbildung der Erfindung ist der Durchmesser D' der distalen Spiralfeder 5 möglichst gross gewählt, aber doch so, dass die Aussenseite der Spiralfeder 5 in der hier gezeigten seitlich versetzten Lage aussenseitig nicht vorstehen kann, was eine optimal glatte Aussenseite im Bereich der Verbindungsstelle ergibt.

## Patentansprüche

1. Verfahren zur Herstellung eines Führungsdrahtes, insbesondere zum perkutanen Einführen eines Ballondilatationskatheters in ein Blutgefäss, welcher Führungsdraht (1) eine flexible Spirale (2) aufweist, die einen ebenfalls flexiblen Schaft (3) umgibt und aus wenigstens zwei Spiralfedern (4, 5) hergestellt ist, die an einer Verbindungsstelle (8) über mehrere Windungen (4a, 5a) zusammengedreht und miteinander verlötet sind, dadurch gekennzeichnet, dass die beiden Spiralfedern (4, 5) jeweils an dem zu verbindenden Ende (4b, 5b) auf eine Zentrierform (7) aufgeschoben, zusammengedreht und verlötet werden und dass anschliessend die Zentrierform (7) wieder entfernt wird.

2. Verfahren nach Anspruch 1, dadurch gekennzeichnet, dass die Zentrierform so ausgebildet ist, dass es das Lot (6) während des Verlötens der Spiralfedern (4, 5) nicht annimmt und sich mit diesem nicht verbindet.

3. Verfahren nach Anspruch 1 oder 2, dadurch gekennzeichnet, dass die Zentrierform (7) aus einer Titan- Legierung, vorzugsweise Nickel-Titan-Legierung besteht.

4. Verfahren nach Anspruch 3, dadurch gekennzeichnet, dass die Legierung Tynel ist.

5. Verfahren nach einem der Ansprüche 1 bis 4, dadurch gekennzeichnet, dass die Zentrierform (7) einen Aussendurchmesser (A) aufweist, der im wesentlichen gleich dem Innendurchmesser (B) wenigstens einer der Spiralfedern (4, 5) ist.

6. Verfahren nach einem der Ansprüche 1 bis 5, dadurch gekennzeichnet, dass die zu verbindenden Windungen (4a, 5a) vor dem Zusammendrehen geöffnet werden.

7. Verfahren nach einem der Ansprüche 1 bis 6, dadurch gekennzeichnet, dass die distale Spiralfeder (5) aus einem röntgensichtbaren Werkstoff, beispielsweise Wolfram und die proximale Spiralfeder (4) aus einem im wesentlichen röntgenunsichtbaren Werkstoff, insbesondere rostfreiem Stahl, hergestellt sind.

8. Führungsdraht, insbesondere zum perkutanen Einführen eines Ballondilatationskatheters in ein Blutgefäss, mit einer flexiblen Spirale (2), die einen ebenfalls flexiblen Schaft (3) umgibt und wenigstens eine distale und eine proximale Spiralfeder (5, 4) aufweist, welche Spiralfedern (4, 5) an einer Verbindungsstelle (8) über mehrere Windungen (4, 5) miteinander verlötet sind, wobei die Spirale (2) an der Verbindungsstelle (8) rohrförmig und mit Ausnahme des genannten Schaftes (3) innen hohl ausgebildet ist, dadurch gekennzeichnet, dass die Spiralfedern (4, 5) an der Verbindungsstelle (8) über mehrere Windungen ineinander zusammengedreht sind, wobei die Spirale (2) an der Verbindungsstelle (8) durchgehend und stufenlos rohrförmig ausgebildet ist.

9. Führungsdraht nach Anspruch 8, dadurch gekennzeichnet, dass das Lot (6) im Bereich der Verbindungsstelle (8) die Aussen- und die Innenseite der Spirale (2) nicht oder nicht wesentlich überragt.

10. Führungsdraht nach Anspruch 8 oder 9, dadurch gekennzeichnet, dass die distale Spiralfeder (5) aus einem Draht besteht, der einen kleineren Durchmesser aufweist als der Draht der proximalen Spiralfeder (4).

## Claims

1. Process for producing a guide wire, in particular for the percutaneous introduction of a balloon dilation catheter into a blood vessel, which guide wire (1) has a flexible coil (2) which surrounds a likewise flexible shaft (3) and is produced from at least two coil springs (4, 5) which are twisted together over several turns (4a, 5a) at a connection location (8) and are soldered to one another, characterized in that at the end to be connected (4b, 5b) the two coil springs (4, 5) are each slid onto a centring mould (7), twisted together and soldered and in that the centring mould (7) is then removed again.

2. Process according to Claim 1, characterized in that the centring mould is designed such that it does not accept the solder (6) during the soldering of the coil springs (4, 5) and does not become joined to it.

3. Process according to Claim 1 or 2, characterized in that the centring mould (7) consists of a titanium alloy, preferably nickel-titanium alloy.

4. Process according to Claim 3, characterized in that the alloy is Tynel.

5. Process according to one of Claims 1 to 4, characterized in that the centring mould (7) has an external diameter (A) which is substantially equal to the internal diameter (B) of at least one of the coil springs (4, 5).

6. Process according to one of Claims 1 to 5, characterized in that the turns (4a, 5a) to be connected are opened before being twisted together.

7. Process according to one of Claims 1 to 6, characterized in that the distal coil spring (5) is made from a material which is visible to X-rays, for example tungsten, and the proximal coil spring (4) is made from a material which is substantially invisible to X-rays, in particular stainless steel.

8. Guide wire, in particular for the percutaneous introduction of a balloon dilation catheter into a blood vessel, with a flexible coil (2) which surrounds a likewise flexible shaft (3) and has at least one distal and one proximal coil spring (5, 4), which coil springs (4, 5) are soldered together over several turns (4, 5) at a connection location (8), wherein the coil (2) is designed to be tubular at the connection location (8) and, with the exception of the said shaft (3), internally hollow, characterized in that the coil springs (4, 5) are twisted together into each other over several turns at the connection location (8), wherein the coil (2) is designed to be continuous and steplessly tubular at the connection location (8).

9. Guide wire according to Claim 8, characterized in that the solder (6) does not, or substantially does not, project beyond the outside and the inside of the coil (2) in the region of the connection location (8).

10. Guide wire according to Claim 8 or 9, characterized in that the distal coil spring (5) consists of a wire which has a smaller diameter than the wire of the proximal coil spring (4).

## Revendications

1. Procédé pour fabriquer un fil de guidage, notamment pour l'introduction percutanée d'un cathéter de dilatation à ballonnet dans un vaisseau sanguin, lequel fil de guidage (1) possède un élément hélicoïdal flexible (2), qui entoure une tige également flexible (3) et est constitué par au moins deux ressorts hélicoïdaux (4, 5), qui sont réunis par enroulement conjoint et sont fixés entre eux par brasage au niveau d'une zone de jonction (8) sur plusieurs spires (4a, 5a), caractérisé en ce que l'on emmanche les deux ressorts hélicoïdaux (4, 5) respectivement à l'extrémité (4b, 5b) à réunir, sur un mandrin de centrage (7), qu'on les réunit par enroulement conjoint et qu'on les fixe par brasage et qu'ensuite on retire alors le mandrin de centrage (7).

2. Procédé selon la revendication 1, caractérisé en ce que le mandrin de centrage est agencé de telle sorte qu'il ne reçoit pas la brasure (6) pendant le brasage des ressorts hélicoïdaux (4, 5) et n'est pas relié à cette brasure.

3. Procédé selon la revendication 1 ou 2, caractérisé en ce que le mandrin de centrage (7) est constitué par un alliage de titane, de préférence un alliage de nickel-titane.

4. Procédé selon la revendication 3, caractérisé en ce que l'alliage est du Tynel.

5. Procédé selon l'une des revendications 1 à 4, caractérisé en ce que le mandrin de centrage (7) possède un diamètre extérieur (A), qui est sensiblement égal au diamètre intérieur (A) d'au moins l'un des ressorts hélicoïdaux (4, 5).

6. Procédé selon l'une des revendications 1 à 5, caractérisé en ce qu'on ouvre les spires (4a, 5b) devant être réunies, avant de les enrouler ensemble.

7. Procédé selon l'une des revendications 1 à 6, caractérisé en ce que le ressort hélicoïdal distal (5) est formé d'un matériau visible par radiographie, par exemple du tungstène et que le ressort hélicoïdal proximal (4) est réalisé en un matériau sensiblement invisible par radiographie, notamment de l'acier inoxydable.

8. Fil de guidage, notamment pour l'introduction percutanée d'un cathéter de dilatation à ballonnet, dans un vaisseau sanguin, comportant un élément hélicoïdal flexible (2), qui entoure une tige également flexible (3) et comporte au moins un ressort hélicoïdal distal et un ressort hélicoïdal proximal (5, 4), lesquels ressorts hélicoïdaux (4, 5) sont réunis par brasage au niveau d'une zone de jonction (8), sur plusieurs spires (4, 5), l'élément hélicoïdal (2) étant réalisé avec une forme tubulaire au niveau de la zone de jonction (8) et étant agencé de manière à être évidé intérieurement à l'exception de ladite tige (3), caractérisé en ce que les ressorts hélicoïdaux (4, 5) sont enroulés l'un dans l'autre par rotation sur plusieurs spires, au niveau de la zone de jonction (8), l'élément hélicoïdal (2) étant agencé avec une forme tubulaire continue et étagée au niveau de la zone de jonction (8).

9. Fil de guidage selon la revendication 8, caractérisé en ce que la brasure (6) ne fait pas saillie ou ne fait pas saillie seulement de façon importante sur la face extérieure et la face intérieure de l'élément hélicoïdal (2) au niveau de la zone de jonction (8).

10. Fil de guidage selon la revendication 8 ou 9, caractérisé en ce que le ressort hélicoïdal distal (5) est formé d'un fil qui possède un diamètre inférieur à celui du fil du ressort hélicoïdal proximal (4).
